# EUROPEAN PATENT SPECIFICATION

(11) **EP 3 274 049 B1**
(45) Date of publication and mention of the grant of the patent: **02.09.2020**
(21) Application number: 16713754.6
(22) Date of filing: 16.03.2016
(51) Int. Cl.: A61N 7/02, A61B 5/01, A61B 5/055, G01R 33/48, A61N 7/00, A61B 5/00, A61B 18/00, A61B 90/00, A61B 17/00

(54) **MEDICAL INSTRUMENT FOR SONICATING A SET OF TARGET VOLUMES**
MEDIZINISCHES INSTRUMENT ZUR BESCHALLUNG EINES SATZES VON ZIELVOLUMEN
INSTRUMENT MÉDICAL DESTINÉ À LA SONICATION D'UN ENSEMBLE DE VOLUMES CIBLES

(30) Priority: 27.03.2015 EP 15161453
(43) Date of publication of application: 31.01.2018
(73) Proprietor: Profound Medical Inc., Mississauga, ON L4W 5K5 (CA)
(72) Inventor: TILLANDER, Matti Oskari, 5656 AE Eindhoven (NL)
(74) Representative: DTS Patent- und Rechtsanwälte Schnekenbühl und Partner mbB
(86) International application number: PCT/EP2016/055616
(87) International publication number: WO 2016/156036

(56) References cited:
- EP-B1- 1 349 612
- WO-A1-2014/067844
- US-A1- 2015 080 705
- PAYNE A1 ET AL: "Minimisation of HIFU pulse heating and interpulse cooling times", INTERNATIONAL JOURNAL OF HYPERTHERMIA, BASINGSTOKE, GB, vol. 26, no. 2, 1 January 2010 (2010-01-01), pages 198-208, XP008170322, ISSN: 0265-6736, DOI: 10.3109/02656730903436459
- JOSHUA COON ET AL: "HIFU treatment time reduction in superficial tumours through focal zone path selection", INTERNATIONAL JOURNAL OF HYPERTHERMIA, BASINGSTOKE, GB, vol. 27, no. 5, 1 August 2011 (2011-08-01) , pages 465-481, XP008170207, ISSN: 0265-6736, DOI: 10.3109/02656736.2011.564597

## Description

### TECHNICAL FIELD OF THE INVENTION

The invention relates to high intensity focused ultrasound devices, in particular it relates to the control of high intensity focused ultrasound devices using magnetic resonance imaging thermometry.

### BACKGROUND OF THE INVENTION

Ultrasound from a focused ultrasonic transducer can be used to selectively treat regions within the interior of the body. Ultrasonic waves are transmitted as high energy mechanical vibrations. These vibrations induce tissue heating as they are damped, and they can also lead to cavitation. Both tissue heating and cavitation can be used to destroy or heat tissue in a clinical setting. However, heating tissue with ultrasound is easier to control than cavitation. Ultrasonic treatments can be used to ablate tissue and to kill regions of cancer cells selectively. This technique has been applied to the treatment of uterine fibroids, and has reduced the need for hysterectomy procedures.

To selectively treat tissue, a focused ultrasonic transducer can be used to focus the ultrasound on a particular treatment or target volume. The transducer is typically mounted within a medium, such as degassed water, that is able to transmit ultrasound. Actuators are then used to adjust the position of the ultrasonic transducer and thereby adjust the tissue region that is being treated.

Focused ultrasonic transducers also typically have multiple transducer elements, wherein the amplitude and/or phase of the transducer elements arc controllable. In particular the phase of individual or groups of transducer elements is often controlled to control the location of the focus of the ultrasound. This enables the rapid adjustment of the location of the focus and the sequential sonication of different sonication points or locations. The tissue of a subject between the transducer and a sonication point is typically referred to as the near field region. The ultrasound travels through the near field region to the sonication volume. This intermediate tissue is also heated, although not as much as the sonication volume. When sonicating multiple sonication points the near field region of the different sonication points may overlap. Because a particular portion of the near field region may overlap it may be heated multiple times. To avoid overheating this overlapping near field region there may need to be delays between sonicating multiple sonication points and/or a reduction in sonication power. European patent application publication EP 2 676 702 A1 discloses a medical instrument comprising a magnetic resonance imaging system and a high intensity focused ultrasound system with an adjustable focus. Execution of instructions causes a processor to control medical instrument to sonicate the multiple sonication points while repeatedly acquire the thermal magnetic resonance imaging data. Multiple thermal maps are reconstructed using the thermal magnetic resonance imaging data and a heating center of mass is calculated for each. By comparing each of the heating center of masses to the sonication points a spatially dependent targeting correction is determined. The spatially dependent targeting correction is then used to offset the adjustable focus.

US Patent Application Publication No. 2015/0080705 discloses a medical instrument comprising a magnetic resonance imaging system and a high-intensity focused ultrasound system with an electronically controllable and a mechanically controllable focus. Execution of instructions by a processor controlling the instrument cause the processor to: receive a target zone descriptive of a zone within the subject; divide the target zone into multiple sub-zones; determine a sequence for moving the transducer position to each of the multiple sub-zones; determine a selected sub-zone selected from the multiple sub-zones using the sequence; repeatedly control the mechanical positioning system to move the transducer to the transducer position of the selected sub-zone; repeatedly acquire the magnetic resonance thermometry data; repeatedly determine a temperature property map; repeatedly heat the regions independently to the target temperature by controlling the electronically controlled focus with a temperature feedback algorithm; and repeatedly change the selected sub-zone using the sequence.

International Patent Application Publication No. WO 2014/067844 discloses a medical apparatus comprising a high intensity focused ultrasound system. Execution of instructions cause a processor to: receive previous sonication data descriptive of a previous sonication of the subject; construct a thermal property map of the subject using the previous sonication data and a thermoacoustic model; determine a maximum energy map using the thermoacoustic model and the thermal property map, wherein the maximum energy is time dependent; display the maximum energy map on a display; and receive a selection of at least one sonication volume from a user interface.

European Patent EP 1 349 61 2 B1 discloses a thermal treatment system including a heat applying element for generating thermal doses for ablating a target mass in a patient. A planner automatically constructs a treatment plan based on input information including one or more of a volume of the target mass, a distance from a skin surface of the patient to the target mass, a set of default thermal dose prediction properties, a set of user specified thermal dose prediction properties, physical properties of the heat applying elements, and images provided by the imager. The default thermal dose prediction properties are preferably based on a type of clinical application and include at least one of thermal dose threshold, thermal dose prediction algorithm, maximum allowed energy for each thermal dose, thermal dose duration for each treatment site, cooling time between thermal doses, and electrical properties for the heat applying element. The user specified thermal dose prediction properties preferably include at least one or more of overrides for any default thermal dose prediction properties, treatment site grid density; and thermal dose prediction properties not specified as default thermal dose prediction properties from the group comprised of thermal dose threshold, thermal dose prediction algorithm, maximum allowed energy for each thermal dose, thermal dose duration for each treatment site cooling time between thermal doses, and electrical properties for the heat applying element.

Payne A. et al., "Minimisation of HIFU pulse heating and interpulse cooling times," International Journal of Hyperthermia, Basingstoke, GB, vol. 26, no. 2, 1 January 2010 (2010-01-01), pages 198-208, presents results from an optimization technique that reduces HIFU treatment times by minimizing individual heating and interpulse cooling times while adhering to normal tissue constraint limits at each sonication position. According to the paper, the potential clinical usefulness is demonstrated through its implementation in three dimensional (3D) simulations of HIFU treatments for a range of tumor geometries. In addition, use of an increased applied power decreased the treatment time. According to the paper, the power maximization and pulse time minimization procedures can be applied independently of the optimization of the focal zone's scan path, size and shape.

Coon et al., "HIFU Treatment Time Reduction in Superficial Tumours Through Focal Zone Path Selection International Journal of Hyperthermia," International Journal of Hyperthermia, Basingstoke, GB, vol. 27, no. 5, 1 August 2011 (2011-08-01), pages 465-481, discloses a study that evaluates the hypothesis that optimizing the path of a high intensity focused ultrasound (HIFU) treatment's N focal zone heating pulses can significantly reduce treatment time, and identifies the underlying bio-thermal principles. According to the paper, thirty-one scanning paths were investigated using 3D simulations, with a minimum thermal dose delivered to every tumor position. Treatment time was calculated as the sum of the N, individually optimized heating and cooling periods. According to the paper, the best paths significantly reduced treatment times, with the largest gains occurring when (1) temperature superposition inside the tumor was maximized by successively heating the focal zone positions located in a 'stack' along the transducer's axis, and (2) the focal zone was moved laterally to an optimized location and another stack was applied. Reduced tumor heating times also reduced energy deposition in the normal tissues, thus reducing or eliminating the need for inter-pulse cooling. According to the paper, further reductions are obtained by correct choice of the transverse scan path.

### SUMMARY OF THE INVENTION

The invention provides for a medical instrument and a computer program product in the independent claims. Embodiments are given in the dependent claims.

As will be appreciated by one skilled in the art, aspects of the present invention may be embodied as an apparatus or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD), Digital Versatile Disks (DVD), and Blu-Ray Disc (BD), for example CD-ROM, CD-RW, CD-R, DVD-ROM, DVD-RW, DVD-R, BD-R, or BD-RE disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further under stood that, when not mutually exclusive, blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical image data is defined herein as two or three dimensional data that has been acquired using a medical imaging scanner. A medical imaging scanner is defined herein as an apparatus adapted for acquiring information about the physical structure of a patient and construct sets of two dimensional or three dimensional medical image data. Medical image data can be used to construct visualizations which are useful for diagnosis by a physician. This visualization can be performed using a computer.

Magnetic Resonance (MR) data is defined herein as being the recorded measurements of radio frequency signals emitted by atomic spins by the antenna of a Magnetic resonance apparatus during a magnetic resonance imaging scan. Magnetic resonance data is an example of medical image data. A Magnetic Resonance Imaging (MRI) image is defined herein as being the reconstructed two or three dimensional visualization of anatomic data contained within the magnetic resonance imaging data. This visualization can be performed using a computer.

Magnetic resonance data may comprise the measurements of radio frequency signals emitted by atomic spins by the antenna of a Magnetic resonance apparatus during a magnetic resonance imaging scan. Magnetic resonance data may contain different types of information which can be derived from it, for example it may contains information which may be used for magnetic resonance thermometry. Magnetic resonance data that may be used for a magnetic resonance imaging thermometry protocol is referred to herein as thermal magnetic resonance data. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonance frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonance frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods.

An 'ultrasound window' as used herein encompasses a window which is effectively transparent to ultrasonic waves or energy. Typically a thin film or membrane is used as an ultrasound window. The ultrasound window may for example be made of a thin membrane of BoPET (Biaxially-oriented polyethylene terephthalate).

In one aspect the invention provides for a medical instrument comprising a high-intensity focused ultrasound system comprising an ultrasonic transducer. The ultrasonic transducer comprises multiple transducer elements for sonicating a target zone. The high-intensity focused ultrasound system is operable for electronically steering a sonication location by controlling the supply of electrical power to each of the multiple transducer elements. The medical instrument further comprises a magnetic resonance imaging system for acquiring thermal magnetic resonance imaging data from an imaging zone. The target zone is within the imaging zone. The medical instrument further comprises a processor or controller for controlling the medical instrument.

The medical instrument further comprises a memory containing machine-executable instructions and thermometry pulse sequence commands. Pulse sequence commands as used herein comprise commands or data which may be converted into commands which are used for controlling the operation of a magnetic resonance imaging system to acquire magnetic resonance data. Often pulse sequences are represented as timing diagrams. The thermometry pulse sequence commands could also be in the form of a timing diagram or Meta data or data descriptive of a timing diagram which are converted into commands for controlling the magnetic resonance imaging system. The thermometry pulse sequence commands cause the magnetic resonance imaging system to acquire the thermal magnetic resonance imaging data according to a magnetic resonance imaging thermometry protocol.

Execution of the instructions causes the processor to receive sonication commands. The sonication commands specify a set of multiple target volumes within the target zone. The multiple target volumes may be considered to be multiple points which are sonicated by the ultrasonic transducer. The sonication commands could be received in a variety of different ways. In one instance they are received from a graphical user interface. For example a physician or other operator could enter the sonication commands into a user interface or graphical user interface. In another example the sonication commands may be received from a hard drive or other computer storage device which the processor is connected to. In other examples the sonication commands could be received from another computing or computer device.

Execution of the instructions further cause the processor to receive a selection of a current target volume selected from the set of multiple target volumes. In some instances this may be specified within the sonication commands. In other instances the selection of the current target volume may for instance be made in response to data received from a user interface. In another example a computer module or program may select the current target volume. The current target volume may be thought of as being equivalent to the first sonication point that is sonicated by the high-intensity focused ultrasound system.

Execution of the machine-executable instructions further cause the processor to repeatedly acquire the thermal magnetic resonance data by controlling the magnetic resonance imaging system with the thermometry pulse sequence commands. Execution of the machine-executable instructions further cause the processor to repeatedly calculate a temperature map using the thermal magnetic resonance data. Execution of the machine-executable instructions further cause the processor to repeatedly control the high-intensity focused ultrasound system to sonicate the current target volume by electronically steering the sonication location to the current target volume. Execution of the machine-executable instructions further cause the processor to repeatedly remove the current target volume from the set of multiple target volumes after controlling the high-intensity focused ultrasound system to sonicate the current target volume.

Execution of the machine-executable instructions further cause the processor to repeatedly calculate a sonication energy for each of the multiple target volumes by using the temperature map. The sonication energy is the total amount of energy which needs to be deposited at each of the remaining of the set of multiple target volumes. In this step essentially each of the remaining sonication points is classified by how much energy it will require to finish the sonication of that particular sonication point. This may be done in different ways with the temperature map. In some instances the current temperature may be used to determine the remaining energy. In other examples the thermal dose may be the important parameter. In this case for example as the temperature map is repeatedly calculated a thermal dose could be calculated for each of the multiple target volumes.

Execution of the machine-executable instructions further cause the processor to select a next target volume from the multiple target volumes using the calculation of the sonication energy for each of the multiple target volumes. The selection of the next target volume comprises searching for the sonication energy with a minimum value.

Execution of the machine-executable instructions further causes the processor to repeatedly set the next target volume as the current target volume. When the next target volume has been set to the current target volume then the processor goes through and repeats the loops of steps again until all of the multiple target volumes have been sonicated.

This embodiment may have the benefit that by selecting the next sonication point in this way the energy used to sonicate the first or subsequent sonication points may be helpful in completing the sonication of other sonication points. This may be used to reduce the total amount of energy which needs to be deposited into a subject to complete the overall sonication of the target zone. As the amount of total energy deposited into the subject is reduced this may have the beneficial effect in some examples of reducing the amount of near field heating of the subject. Also by choosing the method that reduces the total amount of heat deposited or energy, this may reduce the amount of time needed for the sonication. In other examples it may further have the benefit of reducing the amount of time which is required for the subject to cool in between sonications of different target volumes.

In another embodiment execution of the machine-executable instructions further cause the processor to calculate an estimated near field temperature map for each of the multiple target volumes using the temperature map and an ultrasonic transducer module. Execution of the machine-executable instructions further cause the processor to select the next target volume at least partially using the estimated near field temperature map for each of the multiple target volumes. In this example instead of purely going through and calculating the sonication energy the sonications of each of the remaining multiple target volumes is modeled using the ultrasonic transducer module such that an estimated near field temperature map is created for each one.

The benefit of doing this may be that it is possible to predict how hot the near field region of the subject will become. This can then also be used to select which will be the next target volume. This for instance may be useful in reducing the amount of near field heating of the subject such that the subject is not burned or overheated in the near field region. This may be useful in reducing the amount of time which is required for the subject to cool before proceeding to the next target volume. As this is done also using the minimum sonication energy this may be useful in further reducing the possible heating of the near field.

In another embodiment the selecting of the next target volume at least partially using the estimated near field temperature map for each of the multiple target volumes comprises searching the estimated near field temperature map for a high temperature zone which has a temperature above a predetermined threshold. This may be beneficial because it may be able to predict if the near field region of the subject will become overheated if a particular volume is selected as the next target volume. This may be used to selectively exclude particular target volumes from being selected such that is it not necessary to wait for the subject to cool before proceeding with the sonication.

In another embodiment the selecting of the next target volume at least partially using the estimated near field temperature map for each of the multiple target volumes comprises excluding a chosen target volume from being selected as the next target volume if the high temperature zone is found.

In another embodiment selecting the next target volume at least partially using the estimated near field temperature map for each of the multiple target volumes comprises modifying the sonication commands to shut off transducer elements selected from multiple transducer elements that contribute to the heating of the high temperature zone. In this embodiment individual transducer elements which may contribute to heating the high temperature zone can be shut off selectively. This may have the benefit that it reduces the heating of the high temperature zone such that a target volume which had to be ignored for the selection of the next target volume because it caused excessive near field heating can instead be selected. This may contribute further to reducing the amount of delay which is necessary before a subject can be sonicated. This may also have the potential benefit of reducing the overall energy which is needed to complete the total heating of the target zone.

In another embodiment the magnetic resonance imaging thermometry protocol is a proton resonance frequency shift magnetic resonance protocol. The memory further contains calibration pulse sequence commands. The calibration pulse sequence commands cause the magnetic resonance imaging system to acquire phase calibration magnetic resonance data according to the magnetic resonance imaging thermometry protocol. Execution of the machine-executable instructions further cause the processor to acquire the phase calibration magnetic resonance data by controlling the magnetic resonance imaging system with the calibration pulse sequence commands before controlling the high-intensity focused ultrasound system to sonicate the current target volume. Execution of the machine-executable instructions further causes the processor to calculate a phase calibration using the phase calibration magnetic resonance data. The temperature map is calculated using the thermal magnetic resonance data and the phase calibration.

In another embodiment the medical instrument further comprises an actuator system for moving the ultrasonic transducer. The sonication commands specify an actuator position for each of the set of multiple target volumes. Execution of the instructions further cause the processor to create a list of possible actuator positions from the actuator position for each of the set of multiple target volumes. The phase calibration magnetic resonance data is acquired by acquiring the phase calibration magnetic resonance data for each actuator position in the list of possible actuator positions. The phase calibration calculated by calculating the phase calibration for each actuator position is in the list of possible actuator positions. If in a high-intensity focused ultrasound system that has an actuator moving the ultrasonic transducer to different positions may have a large effect on the B0 magnetic field. This embodiment may have the benefit that by performing the calibration at the possible actuator positions an accurate calibration for the PRFS temperature method may be acquired for each of these possible locations. This may greater improve the quality of the temperature measurements made during heating of the subject.

In another embodiment execution of the machine-executable instructions cause the processor to calculate the temperature map for each actuator position in the list of possible actuator positions using the thermal magnetic resonance data for each actuator position in the list of possible actuator positions. Controlling the high-intensity focused ultrasound system to sonicate the current target comprises controlling the actuator system to move the ultrasound transducer to the actuator position of the current target volume.

Using an actuator to move the ultrasound transducer to different physical locations may also be considered a form of steering the high-intensity focused ultrasound system to sonicate the current target volume.

In another aspect the disclosure provides for a method, not forming part of the invention, of operating a medical instrument. The medical instrument comprises a high-intensity focused ultrasound system comprising an ultrasonic transducer. The ultrasonic transducer comprises multiple transducer elements for sonicating a target zone. The high-intensity focused ultrasound system is operable for electronically steering a sonication location by controlling the supply of electrical power to each of the multiple transducer elements. The medical instrument further comprises a magnetic resonance imaging system for acquiring the thermal magnetic resonance imaging data from an imaging zone. The target zone is within the imaging zone.

The method comprises receiving sonication commands. The sonication commands specify a set of multiple target volumes within the target zone. The method further comprises receiving a selection of a current target volume selected from the set of multiple target volumes. The method further comprises repeatedly acquiring the thermal magnetic resonance data by controlling the magnetic resonance imaging system with the thermometry pulse sequence commands. The thermometry pulse sequence commands cause the magnetic resonance imaging system to acquire the thermal magnetic resonance imaging data according to a magnetic resonance imaging thermometry protocol. The method further comprises repeatedly calculating a temperature map using the thermal magnetic resonance data.

The method further comprises repeatedly controlling the high-intensity focused ultrasound system to sonicate the current target volume by steering the sonication location to the current target volume. This may include electronically steering the sonication location and in some instances also physically moving the ultrasonic transducer to different locations. The method further comprises removing the current target volume from the set of multiple target volumes after controlling the high-intensity focused ultrasound system to sonicate the current target volume.

The method further comprises repeatedly calculating a sonication energy for each of the multiple target volumes by using the temperature map. The method further comprises repeatedly selecting a next target volume from the multiple target volumes using the calculation of the sonication energy for each of the multiple target volumes. The selection of the next target volume comprises searching for the sonication energy with a minimum value. The method further comprises repeatedly setting the next target volume as the current target volume.

In another embodiment the method further comprises calculating an estimated near field temperature for each of the multiple target volumes using the temperature map and the ultrasonic transducer model. The method further comprises repeatedly selecting the next target volume at least partially using the estimated near field temperature map for each of the multiple target volumes.

In another embodiment selecting the next target volume at least partially using the estimated near field temperature map for each of the multiple target volumes comprises searching the estimated near field temperature map for a high temperature zone which has a temperature above a predetermined threshold.

In another embodiment selecting the next target volume at least partially using the estimated near field temperature map for each of the multiple target volumes comprises excluding a chosen target volume from being selected as the next target volume if the high temperature zone is found.

In another embodiment selecting the next target volume at least partially using the estimated near field temperature map for each of the multiple target volumes comprises modifying the sonication commands to shut off transducer elements selected from multiple transducer elements that contribute to the heating of the high temperature zone.

In another embodiment the magnetic resonance imaging thermometry protocol is a proton resonance frequency shift magnetic resonance protocol. The method further comprises acquiring the phase calibration magnetic resonance data by controlling the magnetic resonance imaging system with calibration pulse sequence commands before controlling the high-intensity focused ultrasound system to sonicate the current target volume. The calibration pulse sequence commands cause the magnetic resonance imaging system to acquire phase calibration magnetic resonance data according to the magnetic resonance imaging thermometry protocol. The method further comprises calculating a phase calibration according to the phase calibration magnetic resonance data. The temperature map is calculated using the thermal magnetic resonance data and the phase calibration.

In another aspect the invention provides for a computer program product comprising machine-executable instructions for execution by a processor controlling the medical instrument. The medical instrument comprises a high-intensity focused ultrasound system comprising an ultrasonic transducer. The ultrasonic transducer comprises multiple transducer elements for sonicating a target zone. The high-intensity focused ultrasound system is operable for electronically steering a sonication location by controlling the supply of electrical power to each of the multiple transducer elements. The medical instrument further comprises a magnetic resonance imaging system for acquiring thermal magnetic resonance imaging data from an imaging zone. The target zone is within the imaging zone. Execution of the instructions causes the processor to receive sonication commands. The sonication commands specify a set of multiple target volumes within the target zone. Execution of the machine-executable instructions further cause the processor to receive a selection of a current target volume selected from the set of multiple target volumes.

Execution of the machine-executable instructions further cause the processor to repeatedly acquire the thermal magnetic resonance data by controlling the magnetic resonance imaging system with thermometry pulse sequence commands. The thermometry pulse sequence commands cause the magnetic resonance imaging system to acquire the thermal magnetic resonance imaging data according to a magnetic resonance imaging thermometry protocol. Execution of the machine-executable instructions further causes the processor to repeatedly calculate a thermal map using the thermal magnetic resonance data. Execution of the machine-executable instructions further cause the processor to repeatedly control the high-intensity focused ultrasound system to sonicate the current target volume by electronically steering the sonication location to the current target volume.

Execution of the machine-executable instructions further cause the processor to repeatedly remove the current target volume from the set of multiple target volumes after controlling the high-intensity focused ultrasound system to sonicate the current target volume. Execution of the machine-executable instructions further cause the processor to repeatedly calculate a sonication energy for each of the multiple target volumes by using the temperature map. Execution of the machine-executable instructions further cause the processor to repeatedly select a next target volume from the multiple target volumes using the calculation of the sonication energy for each of the multiple target volumes. The selection of the next target volume comprises searching for the sonication energy with a minimum value. Execution of the machine-executable instructions further causes the processor to repeatedly set the next target volume as the current target volume.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 illustrates an example of a medical instrument;
Fig. 2 shows an enlarged view from Fig. 1;
Fig. 3 shows a flow chart that illustrates a method of operating the medical instrument of Fig. 1;
Fig. 4 shows a flow chart that illustrates a further method of operating the medical instrument of Fig. 1; and
Fig. 5 shows a flow chart that illustrates a further method of operating the medical instrument of Fig. 1;

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

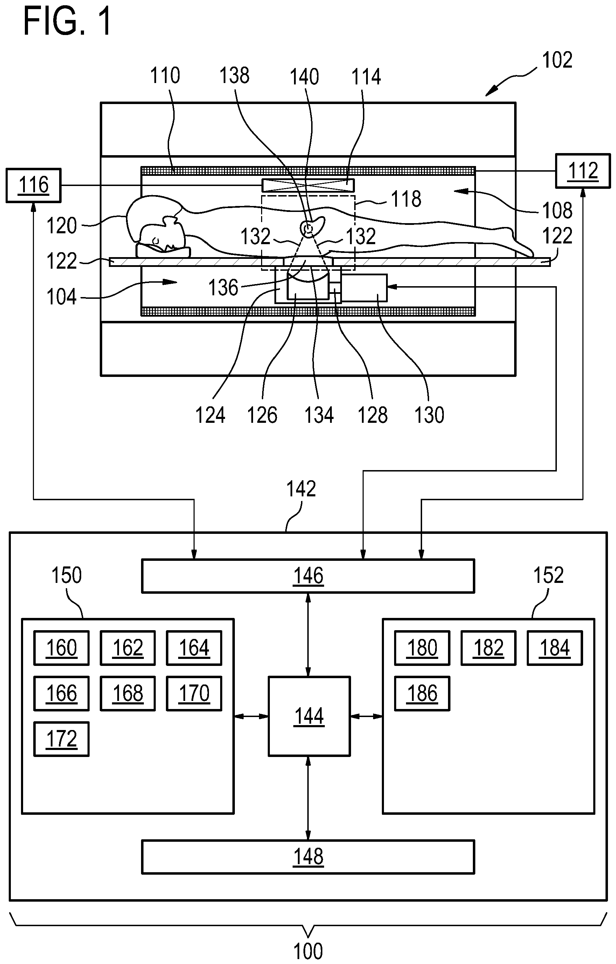

Fig. 1 shows an example of a medical instrument 100. The medical instrument comprises a magnetic resonance imaging system 102 and a high-intensity focused ultrasound system 204. The magnetic resonance imaging system comprises a magnet 106. The magnet shown in Fig. 1 is a cylindrical type superconducting magnet. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 108 of the cylindrical magnet 106 there is an imaging zone 118 where the magnetic field is strong and uniform enough to perform magnetic resonance imaging.

Within the bore 108 of the magnet there is also a magnetic field gradient coil 110 which is used to spatially encode magnetic spins within an imaging zone of the magnet during the acquisition of magnetic resonance data. The magnetic field gradient coil 110 is connected to a magnetic field gradient coil power supply 112. The magnetic field gradient coil is intended to be representative. Typically magnetic field gradient coils contain three separate sets of coils for spatially encoding in three orthogonal spatial directions. A magnetic field gradient power supply supplies current to the magnetic field gradient coils. The current supplied to the magnetic field coils is controlled as a function of time and may be ramped or pulsed.

In the center of the bore 108 is an imaging zone 118. Adjacent to the imaging zone is a radio-frequency coil 114 which is connected to transceiver 116. Also within the bore 108 is a subject 120 reposing on a subject support 122. The radio-frequency coil 114 is adapted for manipulating the orientations of magnetic spins within the imaging zone and for receiving radio transmissions from spins also within the imaging zone. The radio-frequency coil 114 may contain multiple coil elements. The radio-frequency coil may also be referred to as a channel or an antenna. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver may also represent a separate transmitter and receivers.

The high-intensity focused ultrasound system 104 comprises a fluid-filled chamber 124 which houses an ultrasound transducer 126. The ultrasound transducer 126 is mechanically positioned by a mechanical positioning system 128. There is an actuator 130 for actuating the mechanical positioning system. In alternative embodiments the ultrasound transducer may be a manually positioned external transducer without the fluid-filled chamber 124 or mechanical positioning system 128.

The ultrasonic transducer 126 may also contain multiple elements for emitting ultrasound. A power supply which is not shown may control the amplitude and/or phase and/or frequency of alternating current electric power supplied to the elements of the ultrasonic transducer 126. The dashed lines 132 show the path of ultrasound from the ultrasonic transducer 126. The ultrasound 132 first passes through the fluid-filled chamber 124. The ultrasound then passes through an ultrasound window 134. After passing through the ultrasound window 134 the ultrasound passes through an optional gel pad 136 or a layer of ultrasound conductive gel which may be used to conduct ultrasound between the window 134 and the subject 120. The ultrasound 132 then enters the subject 120 and is focused into a focus 138 or sonication point. There is a region 140 which is a target zone. Through a combination of electronic and mechanical positioning of the focus 138 the entire target zone 140 can be heated. The target zone 140 is within the imaging zone 118. The high-intensity focused ultrasound system 104, the transceiver 116, and the magnetic field gradient coil power supply 112 are all connected to a hardware interface 146 of computer system 142. The hardware interface 146 is connected to processor 144. The processor 144 is also connected to a user interface 148, computer storage 150, and computer memory 152.

The computer storage 150 is shown as containing sonication commands 160. The sonication commands could for example been previously stored on the computer storage device 150, could have been entered through the user interface 148, or may have even been received via a network interface of some kind. The computer storage 150 is further shown as containing a selection of a current target volume 162. The selection of the current target volume 162 is where the high-intensity focused ultrasound system 104 focuses the focal point 138. The computer storage 150 is further shown as containing thermometry pulse sequence commands 164. The thermometry pulse sequence commands 164 enable the medical instrument 100 to perform magnetic resonance thermometry. The computer storage 150 is further shown as containing thermal magnetic resonance imaging data 166 that was acquired using the thermometry pulse sequence commands 164.

The computer storage 150 is further shown as containing a temperature map 168 that was reconstructed from the thermal magnetic resonance imaging data 166. The computer storage 150 is further shown as containing a set of target volumes 170 which are used to define where the focal point 138 is placed at different locations within the target zone 140. The computer storage 150 is further shown as containing a map with calculated sonication energies 172. These are the energy which is necessary to complete the sonication of each point in the set of target volumes 170. The calculated sonication energies 172 are used at least partially to select the next target volume that is sonicated.

The computer memory 152 is shown as containing a control module 180. The control module 180 contains computer-executable code which enables the processor 144 to control the operation and function of the various components of the medical instrument 100. The computer storage 152 is further shown as containing an image reconstruction module 182. The image reconstruction module 182 enables the processor 144 to reconstruct magnetic resonance images using magnetic resonance data that is acquired. The image reconstruction module 182 may also contain commands and routines which enable the processor 144 to perform various image processing operations.

The computer storage 152 is further shown as containing a magnetic resonance thermometry module 184. The magnetic resonance thermometry module 184 enables the processor 144 to analyze and process the thermal magnetic resonance imaging data 166 into the temperature map 168. Depending upon the exact magnetic resonance thermometry method used the magnetic resonance thermometry module 184 may also be adapted for performing various types of calibration for magnetic resonance thermometry also. The computer memory 152 is further shown as containing an ultrasound model module 186 which enables the processor 144 to model the ultrasound transducer 126 and any individual transducer elements. This for instance may be useful for performing ray tracing for ultrasound images by various transducer elements. This may be useful in determining which and possibly turning off various transducer elements such that they do not contribute to overheating of the subject's near field zone.

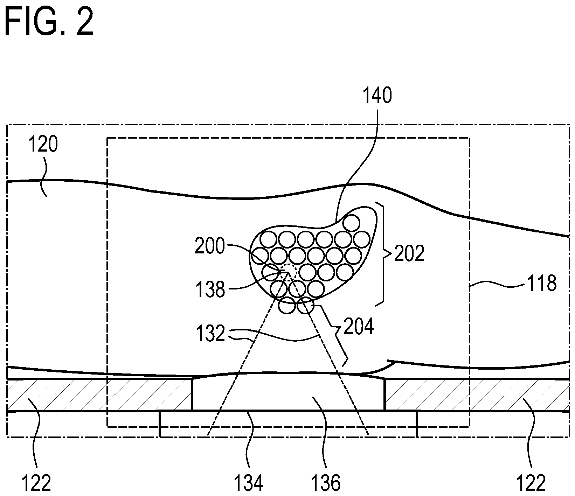

Fig. 2 shows an enlarged view of the imaging zone 118. In particular the target zone 140 can be seen in greater detail. It can be seen that the focal point 138 is focused on a current target volume 200. The target zone is divided into a number of discreet set of target volumes 202. After the location 200 has been sonicated the temperature map is then used to determine which of the remaining target volumes 202 should be sonicated next. This for instance may require electronic and/or mechanical steering of the transducer 126. Between the target volume 202 and the transducer 126 is the near field region 204 of the subject 120. In some examples individual elements of the transducer 126 may be turned off to prevent overheating of the near field region 204.

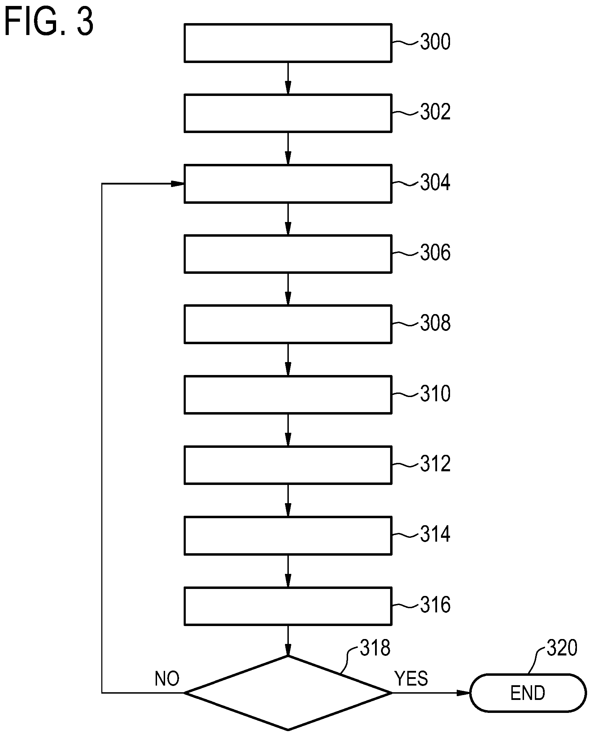

Fig. 3 shows a flowchart which illustrates an example of a method of operating the medical instrument 100 illustrated in Figs. 1 and 2. First in step 300 sonication commands 160 are received. The sonication commands specify a set of target volumes 202 within the target zone 140. Next in step 302 the selection of the current target volume 200 is received. This is the location where the first sonication will be performed. Next in step 304 thermal magnetic resonance data 166 is acquired by controlling the magnetic resonance imaging system 102 with the thermometry pulse sequence commands 164. Next in step 306 a temperature map 168 is calculated using the thermal magnetic resonance imaging data 166. Next in step 308 the high-intensity focused ultrasound system is controlled to sonicate the current target volume 200 by steering the sonication location 138 to the current target volume 200.

Next in step 310 the current target volume 200 is removed from the set of multiple target volumes 270 after controlling the high-intensity focused ultrasound system 104 to sonicate the current target volume 200. In step 312 a sonication energy 172 is calculated for each of the multiple target volumes 202 using the temperature map 168. Next in step 314 a next target volume is selected from the multiple target volumes using the calculation of the sonication energy 172 for each of the multiple target volumes 202. The selection of the next target volume comprises searching for a sonication energy with a minimum value.

Next in step 316 the next target volume is set as the current target volume. 318 is a decision box and the question is, have all target volumes been sonicated. If the answer is yes then the method proceeds to step 320 and the method of Fig. 3 ends. If no, then the method returns back to step 304. It should be noted that the steps shown in Fig. 3, various steps may be re-arranged and placed in a different order. Also during the execution of the method shown in Fig. 3, several of the steps may be performed at the same time. For example the acquisition of the thermal magnetic resonance data may be done continuously and as the data is acquired the various thermal maps and corrections are done as they become available. The sonication of the current target volume may also be done during the execution of some steps. For example the acquisition of the thermal magnetic resonance data and the sonication of the current target volume may be performed at the same time.

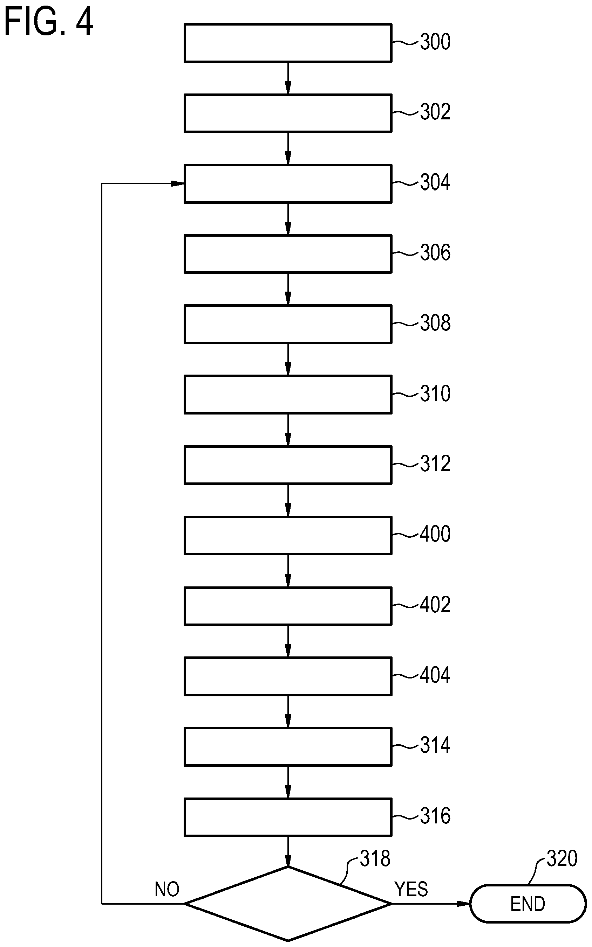

Fig. 4 shows a flowchart which illustrates a further method of operating the medical instrument shown in Figs. 1 and 2. The method shown in Fig. 4 is similar to that in Fig. 3. However, in the method shown in Fig. 4 the method proceeds from step 312 to step 400. In step 400 an estimated near field temperature map is calculated for each of the multiple target volumes 202 using the temperature map 168 and an ultrasonic transducer module model 186. The target volume is then at least partially selected using the near field temperature map for each of the multiple target volumes. After step 400 the method proceeds to step 402. In step 402 the estimated near field temperature maps are searched for a high temperature zone which has a temperature above a predetermined threshold. In other words the near field temperature maps are searched for regions which have a temperature above an allowed or predetermined value. The model is then used to predict regions where there will be overheating of the subject.

The method then proceeds to step 404, where the sonication commands are modified to shut off transducer elements of the ultrasonic transducer 126 that contribute to the hearing of the high temperature zone. In this step high temperature regions that have been identified are then used to modify the sonication commands and individual transducer elements are shut off in an effort to reduce the heating of these high temperature zones. This may be beneficial because it enables the sonication of the various target volumes to proceed with little or no delay. After step 404 the method returns normally to step 314 and the method of Fig. 4 is then identical with the method of Fig. 3.

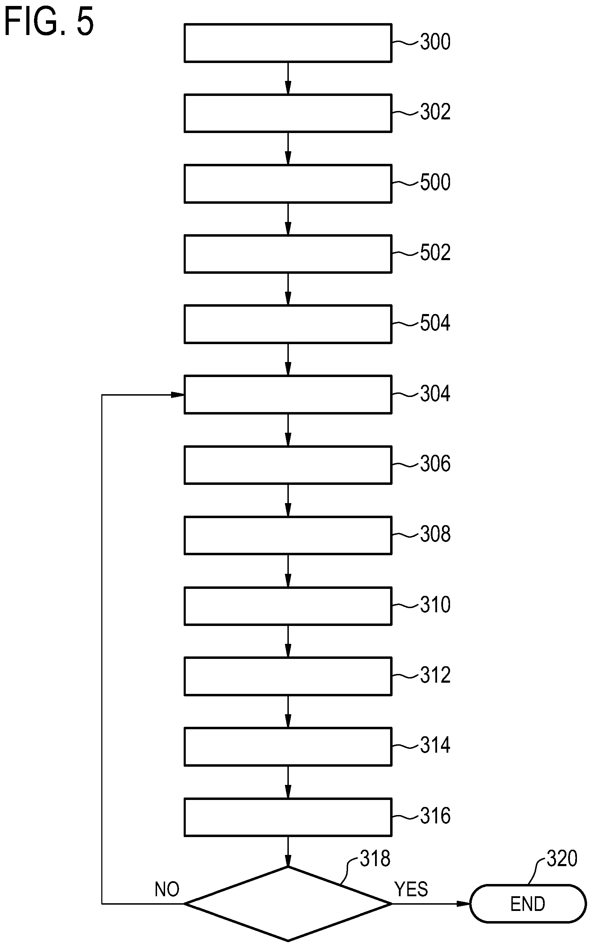

Fig. 5 shows a further example of a flowchart of a method of operating the medical instrument of Figs. 1 and 2. The method of Fig. 5 is similar to that of Fig. 3 with several additions. After step 302 is performed the method proceeds to step 500. In step 500 a list of possible actuator positions of the ultrasound transducer 126 is performed. The sonication commands specify an actuator position for each of the set of multiple target volumes. This is then used to make the list of possible actuator positions. Next in step 502 phase calibration magnetic resonance data is acquired by controlling the magnetic resonance imaging system with calibration pulse sequence commands. The phase calibration magnetic resonance data is acquired for each of the various positions in the list of possible actuator positions.

Finally in step 404, a phase calibration for each of the possible actuator positions is calculated using the phase calibration magnetic resonance data at each of those locations. This enables the temperature map to be calculated using a different calibration for each actuator position that is used when positioning the ultrasonic transducer 126. This may result in more accurate temperature measurements during use of the medical instrument 100.

The steps of Figs. 4 and 5 may be combined.

MR-guided High Intensity Focus Ultrasound (HIFU) therapies that use proton resonance frequency method for monitoring and controlling the heating typically consist of several separate sonications. Each sonication begins with a gathering of reference phase image, after which applying of the ultrasound energy is started. Phase images are gathered during the sonication and used to reconstruct the temperature maps, which are then used to control the heating. After the heating is finished a cooling period is required before the next sonication can be started. This is due to the fact that in focused ultrasound sonications also the near field, i.e. intervening tissues such as skin and fat layer, are inevitably heated. In order to prevent the overheating of near field tissues cooling periods need to be added between sonication. This will make the total treatment time longer and reduces the therapy efficiency. The length of the cooling period depends on the applied energy in the sonication, which is defined by sonication size, duration, and power. Making cooling periods shorter would improve the total therapy efficiency.

Examples may help in reducing the cooling time and thus the chance of overheating the non-targeted tissues and/or increasing the therapy efficiency.

Certain examples may have one or more of the following features:
1. MR temperature mapping that can compensate for the susceptibility artefacts caused the change in the transducer position.
2. Sonication algorithm that can exploit the heat that diffuses from the neighboring sonication targets.
3. Element switch-off algorithm to control the near field heating.

Various example may enable performing sonication cells from multiple sonication positions without a cooling period and new reference image gathering phase between sonications. In the beginning the user may set for example three sonication cells that are close to each other but which require transducer movement in between. Reference images are gathered separately for each cell by moving the transducer to each location one at a time prior starting of the sonication. When the reference images have been gathered the sonication starts from one cell and after it has been treated, the transducer is moved to another position. After movement the temperature mapping needs to change the used reference image that corresponds to the new transducer position.

Since the cells are positioned closely to each other, the heating of the second cell may now exploit the heat that diffuses from the first cell. This reduces the amount of energy needed to heat the second cell and thus increases the heating efficiency respect the near field heating. After the second cell has been treated, the sonication is continued from the next cell. The maximum amount of subsequently treatable cells will be eventually limited by for example tissue perfusion and size of cells. Since the acoustic fields for each position may overlap in the near field causing hot spots, the near field heating may be controlled by using element switch-off. The cells can be positioned for example on a same plane or along the same beam axis. In the latter choice the temperature mapping slices should be moved accordingly to the transducer movement.

While the invention has been illustrated and described in detail in the drawings and foregoing description, such illustration and description are to be considered illustrative or exemplary and not restrictive; the invention is limited only by the appended claims.

Other variations to the disclosed embodiments can be understood and effected by those skilled in the art in practicing the claimed invention, from a study of the drawings, the disclosure, and the appended claims. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: magnetic resonance imaging system
- 104: high-intensity focused ultrasound system
- 106: magnet
- 108: bore of magnet
- 110: magnetic field gradient coil
- 112: magnetic field gradient coil power supply
- 114: radio frequency coil
- 116: transceiver
- 118: imaging zone
- 120: subject
- 122: subject support
- 124: fluid filled chamber
- 126: ultrasonic transducer
- 128: mechanical positioning system
- 130: actuator
- 132: path of ultrasound
- 134: ultrasound window
- 136: gel pad
- 138: focus
- 140: target zone
- 142: computer system
- 144: processor
- 146: hardware interface
- 148: user interface
- 150: computer storage
- 152: computer memory
- 160: sonication commands
- 162: selection of current target volume
- 164: thermometry pulse sequence commands
- 166: thermal magnetic resonance data
- 168: temperature map
- 170: set of target volumes
- 172: calculated sonication energies
- 180: control module
- 182: image reconstruction module
- 184: MR thermometry module
- 186: ultrasound model module
- 200: current target volume
- 202: target volumes
- 204: nearfield region
- 300: receive sonication commands, wherein the sonication commands specify a set of multiple target volumes within the target zone
- 302: receive a selection of a current target volume selected from the set of multiple target volumes
- 304: acquire the thermal magnetic resonance data by controlling the magnetic resonance imaging system with the thermometry pulse sequence commands
- 306: calculate a temperature map using the thermal magnetic resonance data
- 308: control the high intensity focused ultrasound system to sonicate the current target volume by electronically steering the sonication location to the current target volume
- 310: remove the current target volume from the set of multiple target volumes after controlling the high intensity focused ultrasound system to sonicate the current target volume
- 312: calculate a sonication energy for each of the multiple target volumes by using the temperature map
- 314: select a next target volume from the multiple target volumes using the calculation of the sonication energy for each of the multiple target volumes
- 316: set the next target volume as the current target volume
- 318: have all of the multiple target volumes been sonicated?
- 320: end
- 400: calculate an estimated near field temperature map for each of the multiple target volumes using the temperature map and an ultrasonic transducer model
- 402: search the estimated near field temperature map for a high temperature zone which has a temperature above a predetermined threshold
- 404: modify the sonication commands to shut off transducer elements selected from multiple transducer elements that contribute to the heating of the high temperature zone
- 500: create a list of possible actuator positions from the actuator position for each of the set of multiple target volume
- 502: acquire the phase calibration magnetic resonance data for each of the possible actuator positions by controlling the magnetic resonance imaging system with the calibration pulse sequence commands
- 504: calculate a phase calibration according with the phase calibration magnetic resonance data for each of the possible actuator positions

## Claims

1. A medical instrument (100) comprising:
a high intensity focused ultrasound system (104) comprising an ultrasonic transducer; wherein the ultrasonic transducer comprises multiple transducer elements for sonicating a target zone (140), wherein the high intensity focused ultrasound system is operable for electronically steering a sonication location (138) by controlling the supply of electrical power to each of the multiple transducer elements;
a magnetic resonance imaging system (102) for acquiring thermal magnetic resonance imaging data (166) from an imaging zone (118), wherein the target zone is within the imaging zone;
a processor (144) for controlling the medical instrument;
a memory (152) containing machine executable instructions (180, 182, 184, 186) and thermometry pulse sequence commands (164), wherein the thermometry pulse sequence commands cause the magnetic resonance imaging system to acquire the thermal magnetic resonance imaging data according to a magnetic resonance imaging thermometry protocol; wherein execution of the machine executable instructions causes the processor to:
receive (300) sonication commands (160), wherein the sonication commands specify a set of multiple target volumes (202) within the target zone; and
receive (302) a selection of a current target volume (200) selected from the set of multiple target volumes;
wherein execution of the machine executable instructions causes the processor to repeatedly:
acquire (304) the thermal magnetic resonance data by controlling the magnetic resonance imaging system with the thermometry pulse sequence commands;
calculate (306) a temperature map (168) using the thermal magnetic resonance data;
control (308) the high intensity focused ultrasound system to sonicate the current target volume by steering the sonication location to the current target volume;
remove (310) the current target volume from the set of multiple target volumes after controlling the high intensity focused ultrasound system to sonicate the current target volume;
calculate (312) a sonication energy (172) that needs to be deposited at a target volume for each of the multiple target volumes by using the temperature map;
select (314) a next target volume from the multiple target volumes based on the calculated sonication energy, **characterized in that** the next target volume is a target volume, which will require a minimum sonication energy to finish the sonication; and
set (316) the next target volume as the current target volume.

2. The medical instrument of claim 1, wherein execution of the machine executable instructions further causes the processor to repeatedly:
calculate (400) an estimated near field temperature map for each of the multiple target volumes using the temperature map and an ultrasonic transducer model, and
select the next target volume at least partially using the estimated near field temperature map for each of the multiple target volumes.

3. The medical instrument of claim 2, wherein selecting the next target volume at least partially using the estimated near field temperature map for each of the multiple target volumes comprises searching (402) the estimated near field temperature map for a high temperature zone which has a temperature above a predetermined threshold.

4. The medical instrument of claim 3, wherein selecting the next target volume at least partially using the estimated near field temperature map for each of the multiple target volumes comprises excluding a chosen target volume from being selected as the next target volume if the high temperature zone is found.

5. The medical instrument of claim 3 or 4, wherein selecting the next target volume at least partially using the estimated near field temperature map for each of the multiple target volumes comprises modifying (404) the sonication commands to shut off transducer elements selected from multiple transducer elements that contribute to the heating of the high temperature zone.

6. The medical instrument of any one of the preceding claims, wherein the magnetic resonance imaging thermometry protocol is a proton resonance frequency shift magnetic resonance protocol, wherein the memory further contains calibration pulse sequence commands, wherein the calibration pulse sequence commands cause the magnetic resonance imaging system to acquire phase calibration magnetic resonance data according to the magnetic resonance imaging thermometry protocol, wherein execution of the machine executable instructions further causes the processor to:
acquire (502) the phase calibration magnetic resonance data by controlling the magnetic resonance imaging system with the calibration pulse sequence commands before controlling the high intensity focused ultrasound system to sonicate the current target volume, and
calculate (504) a phase calibration using the phase calibration magnetic resonance data, wherein the temperature map is calculated using the thermal magnetic resonance data and the phase calibration.

7. The medical instrument of claim 6, wherein the medical instrument further comprises an actuator system for moving the ultrasonic transducer, wherein the sonication commands specify an actuator position for each of the set of multiple target volumes, wherein execution of the instructions further causes the processor to create (500) a list of possible actuator positions from the actuator position for each of the set of multiple target volumes, wherein the phase calibration magnetic resonance data is acquired by acquiring the phase calibration magnetic resonance data for each actuator position in the list of possible actuator positions, wherein the phase calibration calculated by calculating the phase calibration for each actuator position in the list of possible actuator positions.

8. The medical instrument of claim 7, wherein execution of the machine executable instructions cause the processor to calculate the temperature map for each actuator position in the list of possible actuator positions using the thermal magnetic resonance data for each actuator position in the list of possible actuator positions, wherein controlling the high intensity focused ultrasound system to sonicate the current target comprises controlling the actuator system to move the ultrasonic transducer to the actuator position of the current target volume.

9. A computer program product comprising machine executable instructions (180, 182, 184, 186) for execution by a processor (144) controlling a medical instrument (100), wherein the medical instrument comprises a high intensity focused ultrasound system (104) comprising an ultrasonic transducer; wherein the ultrasonic transducer comprises multiple transducer elements for sonicating a target zone (140), wherein the high intensity focused ultrasound system is operable for electronically steering a sonication location (138) by controlling the supply of electrical power to each of the multiple transducer elements, wherein the medical instrument further comprises a magnetic resonance imaging system (102) for acquiring thermal magnetic resonance imaging data from an imaging zone, wherein the target zone is within the imaging zone,
wherein execution of the machine executable instructions causes the processor to:
receive (300) sonication commands (160), wherein the sonication commands specify a set of multiple target volumes (202) within the target zone; and
receive (302) a selection of a current target volume (200) selected from the set of multiple target volumes;
wherein execution of the machine executable instructions causes the processor to repeatedly:
acquire (304) the thermal magnetic resonance data by controlling the magnetic resonance imaging system with thermometry pulse sequence commands, wherein the thermometry pulse sequence commands cause the magnetic resonance imaging system to acquire the thermal magnetic resonance imaging data according to a magnetic resonance imaging thermometry protocol;
calculate (306) a temperature map (168) using the thermal magnetic resonance data;
control (308) the high intensity focused ultrasound system to sonicate the current target volume by steering the sonication location to the current target volume;
remove (310) the current target volume from the set of multiple target volumes after controlling the high intensity focused ultrasound system to sonicate the current target volume;
calculate (312) a sonication energy (172) for each of the multiple target volumes by using the temperature map;
select (314) a next target volume from the multiple target volumes using the calculation of the sonication energy for each of the multiple target volumes, **characterized in that** the selection of the next target volume comprises searching for the sonication energy with a minimum value; and
set (316) the next target volume as the current target volume.

## Patentansprüche

1. Medizinisches Instrument (100), aufweisend:
ein leistungsstarkes fokussiertes Ultraschallsystem (104) mit einem Ultraschallwandler; wobei der Ultraschallwandler mehrere Wandlerelemente zur Beschallung einer Zielzone (140) aufweist, wobei das leistungsstarke fokussierte Ultraschallsystem so betrieben werden kann, dass durch Steuern der Zufuhr elektrischer Energie zu jedem der mehreren Wandlerelemente ein Beschallungsort (138) elektronisch geführt wird;
ein Kernspintomographiesystem (102) zum Erfassen thermischer Kernspintomographiedaten (166) von einer Abbildungszone (118), wobei sich die Zielzone innerhalb der Abbildungszone befindet;
einen Prozessor (144) zum Steuern des medizinischen Instruments;
einen Speicher (152), der maschinenausführbare Anweisungen (180, 182, 184, 186) und Thermometrieimpulssequenzbefehle (164) enthält, wobei die Thermometrieimpulssequenzbefehle das Kernspintomographiesystem dazu veranlassen, die thermischen Kernspintomographiedaten gemäß einem Kernspintomographie-Thermometrieprotokoll zu erfassen; wobei die Ausführung der maschinenausführbaren Anweisungen den Prozessor zu Folgendem veranlassen:
Empfangen (300) von Beschallungsbefehlen (160), wobei die Beschallungsbefehle einen Satz von mehreren Zielvolumina (202) innerhalb der Zielzone angeben; und
Empfangen (302) einer Auswahl eines aktuellen Zielvolumens (200), das aus dem Satz mehrerer Zielvolumina ausgewählt ist;
wobei die Ausführung der maschinenausführbaren Anweisungen den Prozessor dazu veranlasst, Folgendes wiederholt auszuführen:
Erfassen (304) der thermischen Magnetresonanzdaten durch Steuern des Kernspintomographiesystems mit den Thermometrieimpulssequenzbefehlen;
Berechnen (306) einer Temperaturkarte (168) unter Verwendung der thermischen Magnetresonanzdaten;
Steuern (308) des leistungsstarken fokussierten Ultraschallsystems zum Beschallen des aktuellen Zielvolumens durch Führen des Beschallungsorts zum aktuellen Zielvolumen;
Entfernen (310) des aktuellen Zielvolumens aus dem Satz mehrerer Zielvolumina nach dem Steuern des leistungsstarken fokussierten Ultraschallsystems zum Beschallen des aktuellen Zielvolumens;
Berechnen (312) einer an einem Zielvolumen abzusetzenden Beschallungsenergie (172) für jedes der mehreren Zielvolumina unter Verwendung der Temperaturkarte;
Auswählen (314) eines nächsten Zielvolumens aus den mehreren Zielvolumina beruhend auf der berechneten Beschallungsenergie, **dadurch gekennzeichnet, dass**
das nächste Zielvolumen ein Zielvolumen ist, das eine minimale Beschallungsenergie zum Abschließen der Beschallung benötigt; und
Ansetzen (316) des nächsten Zielvolumens als aktuelles Zielvolumen.

2. Medizinisches Instrument nach Anspruch 1, wobei die Ausführung der maschinenausführbaren Anweisungen darüber hinaus den Prozessor dazu veranlasst, Folgendes wiederholt auszuführen:
Berechnen (400) einer geschätzten Nahfeldtemperaturkarte für jedes der mehreren Zielvolumina unter Verwendung der Temperaturkarte und eines Ultraschallwandlermodells, und
Auswählen des nächsten Zielvolumens zumindest teilweise unter Verwendung der geschätzten Nahfeldtemperaturkarte für jedes der mehreren Zielvolumina.

3. Medizinisches Instrument nach Anspruch 2, wobei das Auswählen des nächsten Zielvolumens zumindest teilweise unter Verwendung der geschätzten Nahfeldtemperaturkarte für jedes der mehreren Zielvolumina umfasst, die geschätzte Nahfeldtemperaturkarte nach einer Hochtemperaturzone zu durchsuchen (402), die eine Temperatur über einem vorbestimmten Schwellenwert hat.

4. Medizinisches Instrument nach Anspruch 3, wobei das Auswählen des nächsten Zielvolumens zumindest teilweise unter Verwendung der geschätzten Nahfeldtemperaturkarte für jedes der mehreren Zielvolumina umfasst, ein gewähltes Zielvolumen von der Auswahl als nächstes Zielvolumen auszuschließen, wenn die Hochtemperaturzone aufgefunden wird.

5. Medizinisches Instrument nach Anspruch 3 oder 4, wobei das Auswählen des nächsten Zielvolumens zumindest teilweise unter Verwendung der geschätzten Nahfeldtemperaturkarte für jedes der mehreren Zielvolumina umfasst, die Beschallungsbefehle zu modifizieren (404), um aus den mehreren Wandlerelementen ausgewählte Wandlerelemente abzuschalten, die zur Erwärmung der Hochtemperaturzone beitragen.

6. Medizinisches Instrument nach einem der vorhergehenden Ansprüche, wobei das Kernspintomographie-Thermometrieprotokoll ein Protonenresonanz-Frequenzverschiebungs-Magnetresonanzprotokoll ist, wobei der Speicher darüber hinaus Kalibrierungsimpulssequenzbefehle enthält, wobei die Kalibrierungsimpulssequenzbefehle das Kernspintomographiesystem dazu veranlassen, Phasenkalibrierungs-Magnetresonanzdaten gemäß dem Kernspintomographie-Thermometrieprotokoll zu erfassen, wobei die Ausführung der maschinenausführbaren Anweisungen darüber hinaus den Prozessor zu Folgendem veranlassen:
Erfassen (502) der Phasenkalibrierungs-Magnetresonanzdaten durch Steuern des Kernspintomographiesystems mit den Kalibrierungsimpulssequenzbefehlen vor dem Steuern des leistungsstarken fokussierten Ultraschallsystems zum Beschallen des aktuellen Zielvolumens, und
Berechnen (504) einer Phasenkalibrierung unter Verwendung der Phasenkalibrierungs-Magnetresonanzdaten, wobei die Temperaturkarte unter Verwendung der thermischen Magnetresonanzdaten und der Phasenkalibrierung berechnet wird.

7. Medizinisches Instrument nach Anspruch 6, wobei das medizinische Instrument darüber hinaus ein Stellantriebssystem zum Bewegen des Ultraschallwandlers aufweist, wobei die Beschallungsbefehle eine Stellantriebsposition für jedes aus dem Satz mehrerer Zielvolumina angibt, wobei die Ausführung der Anweisungen darüber hinaus den Prozessor dazu veranlasst, eine Liste möglicher Stellantriebspositionen von der Stellantriebsposition für jedes aus dem Satz mehrerer Zielvolumina zu erzeugen (500), wobei die Phasenkalibrierungs-Magnetresonanzdaten erfasst werden, indem die Phasenkalibrierungs-Magnetresonanzdaten für jede Stellantriebsposition in der Liste möglicher Stellantriebspositionen erfasst werden, wobei die Phasenkalibrierung berechnet wird, indem die Phasenkalibrierung für jede Stellantriebsposition in der Liste möglicher Stellantriebspositionen berechnet wird.

8. Medizinisches Instrument nach Anspruch 7, wobei die Ausführung der maschinenausführbaren Anweisungen den Prozessor dazu veranlasst, die Temperaturkarte für jede Stellantriebsposition in der Liste möglicher Stellantriebspositionen unter Verwendung der thermischen Magnetresonanzdaten für jede Stellantriebsposition in der Liste möglicher Stellantriebspositionen zu berechnen, wobei das Steuern des leistungsstarken fokussierten Ultraschallsystems zum Beschallen des aktuellen Ziels umfasst, das Stellantriebssystem so zu steuern, um den Ultraschallwandler zur Stellantriebsposition des aktuellen Zielvolumens zu bewegen.

9. Computerprogrammprodukt, das maschinenausführbare Anweisungen (180, 182, 184, 186) zur Ausführung durch einen Prozessor (144) aufweist, der ein medizinisches Instrument (100) steuert, wobei das medizinische Instrument ein leistungsstarkes fokussiertes Ultraschallsystem (104) mit einem Ultraschallwandler aufweist; wobei der Ultraschallwandler mehrere Wandlerelemente zur Beschallung eine Zielzone (140) aufweist, wobei das leistungsstarke fokussierte Ultraschallsystem so betrieben werden kann, dass durch Steuern der Zufuhr elektrischer Energie zu jedem der mehreren Wandlerelemente ein Beschallungsort (138) elektronisch geführt wird, wobei das medizinische Instrument darüber hinaus ein Kernspintomographiesystem (102) zum Erfassen thermischer Kernspintomographiedaten von einer Abbildungszone aufweist, wobei sich die Zielzone innerhalb der Abbildungszone befindet,
wobei die Ausführung der maschinenausführbaren Anweisungen den Prozessor zu Folgendem veranlassen:
Empfangen (300) von Beschallungsbefehlen (160), wobei die Beschallungsbefehle einen Satz von mehreren Zielvolumina (202) innerhalb der Zielzone angeben; und
Empfangen (302) einer Auswahl eines aktuellen Zielvolumens (200), das aus dem Satz mehrerer Zielvolumina ausgewählt ist;
wobei die Ausführung der maschinenausführbaren Anweisungen den Prozessor dazu veranlasst, Folgendes wiederholt auszuführen:
Erfassen (304) der thermischen Magnetresonanzdaten durch Steuern des Kernspintomographiesystems mit Thermometrieimpulssequenzbefehlen, wobei die Thermometrieimpulssequenzbefehle das Kernspintomographiesystem dazu veranlassen, die thermischen Kernspintomographiedaten gemäß einem Kernspintomographie-Thermometrieprotokoll zu erfassen;
Berechnen (306) einer Temperaturkarte (168) unter Verwendung der thermischen Magnetresonanzdaten;
Steuern (308) des leistungsstarken fokussierten Ultraschallsystems zum Beschallen des aktuellen Zielvolumens durch Führen des Beschallungsorts zum aktuellen Zielvolumen;
Entfernen (310) des aktuellen Zielvolumens aus dem Satz mehrerer Zielvolumina nach dem Steuern des leistungsstarken fokussierten Ultraschallsystems zum Beschallen des aktuellen Zielvolumens;
Berechnen (312) einer Beschallungsenergie (172) für jedes der mehreren Zielvolumina unter Verwendung der Temperaturkarte;
Auswählen (314) eines nächsten Zielvolumens aus den mehreren Zielvolumina unter Verwendung der Berechnung der Beschallungsenergie für jedes der mehreren Zielvolumina, **dadurch gekennzeichnet, dass** die Auswahl des nächsten Zielvolumens umfasst, nach der Beschallungsenergie mit einem Mindestwert zu suchen; und
Ansetzen (316) des nächsten Zielvolumens als aktuelles Zielvolumen.

## Revendications

1. Instrument médical (100) comprenant :
un système à ultrasons focalisés à haute intensité (104) comprenant un transducteur ultrasonique ;
dans lequel le transducteur ultrasonique comprend de multiples éléments transducteurs pour traiter par ultrasons une zone cible (140), dans lequel le système à ultrasons focalisés à haute intensité peut être utilisé pour diriger électroniquement un emplacement de traitement par ultrasons (138) en commandant l'amenée de courant électrique vers chacun des multiples éléments transducteurs ;
un système d'imagerie à résonance magnétique (102) pour acquérir des données d'imagerie de résonance magnétique thermique (166) provenant d'une zone d'imagerie (118), dans lequel la zone cible est à l'intérieur de la zone d'imagerie ;
un processeur (144) pour commander l'instrument médical ;
une mémoire (152) contenant des instructions exécutables par machine (180, 182, 184, 186) et des commandes de séquence d'impulsion thermométrique (164), dans lequel les commandes de séquence d'impulsion thermométrique amènent le système d'imagerie à résonance magnétique à acquérir des données d'imagerie de résonance magnétique thermique en fonction d'un protocole de thermométrie d'imagerie par résonance magnétique ; dans lequel l'exécution des instructions exécutables par machine amène le processeur à :
recevoir (300) les commandes de traitement par ultrasons (160), dans lequel les commandes de traitement par ultrasons spécifient un ensemble de multiples volumes cibles (202) à l'intérieur de la zone cible ; et
recevoir (302) une sélection d'un volume cible actuel (200) sélectionné dans le groupe constitué par l'ensemble de multiples volumes cibles ;
dans lequel l'exécution des instructions exécutables par machine amène de façon répétée le processeur à :
acquérir (304) les données de résonance magnétique thermique en commandant le système d'imagerie à résonance magnétique avec les commandes de séquence d'impulsion thermométrique ;
calculer (306) une carte de température (168) à l'aide des données de résonance magnétique thermique ;
commander (308) le système à ultrasons focalisés à haute intensité pour traiter par ultrasons le volume cible actuel en dirigeant l'emplacement de traitement par ultrasons vers le volume cible actuel ;
retirer (310) le volume cible actuel hors de l'ensemble de multiples volumes cibles après commande du système à ultrasons focalisés à haute intensité pour traiter par ultrasons le volume cible actuel ;
calculer (312) une énergie de traitement par ultrasons (172) devant être déposée à un volume cible pour chacun des multiples volumes cibles à l'aide de la carte de température ;
sélectionner (314) un volume cible suivant à partir des multiples volumes cibles sur la base de l'énergie de traitement par ultrasons calculée ;
**caractérisé en ce que** le volume cible suivant est un volume cible qui nécessitera une énergie de traitement par ultrasons minimale pour finir le traitement par ultrasons ; et
définir (316) le volume cible suivant comme le volume cible actuel.

2. Instrument médical selon la revendication 1, dans lequel l'exécution des instructions exécutables par machine amène en outre de façon répétée le processeur à :
calculer (400) une carte de température de champ proche estimée pour chacun des multiples volumes cibles à l'aide de la carte de température et d'un modèle de transducteur ultrasonique ; et
sélectionner le volume cible suivant au moins en partie à l'aide de la carte de température de champ proche estimée pour chacun des multiples volumes cibles.

3. Instrument médical selon la revendication 2, dans lequel la sélection du volume cible suivant au moins en partie à l'aide de la carte de température de champ proche estimée pour chacun des multiples volumes cibles comprend la recherche (402) de la carte de température de champ proche estimée pour une zone de température élevée qui a une température au-dessus d'un seuil prédéterminé.

4. Instrument médical selon la revendication 3, dans lequel la sélection du volume cible suivant au moins en partie à l'aide de la carte de température de champ proche estimée pour chacun des multiples volumes cibles comprend l'exclusion d'un volume cible choisi pour qu'il ne puisse être sélectionné comme volume cible suivant en cas de détection d'une zone de température élevée.

5. Instrument médical selon la revendication 3 ou 4, dans lequel la sélection du volume cible suivant au moins en partie à l'aide de la carte de température de champ proche estimée pour chacun des multiples volumes cibles comprend la modification (404) des commandes de traitement par ultrasons pour déconnecter les éléments transducteurs sélectionnés dans le groupe constitué par les multiples éléments transducteurs contribuant au chauffage de la zone de température élevée.

6. Instrument médical selon l'une quelconque des revendications précédentes, dans lequel le protocole de thermométrie d'imagerie par résonance magnétique est un protocole de résonance magnétique de changement de vitesse de fréquence de résonance de proton, dans lequel la mémoire contient en outre des commandes de séquence d'impulsion d'étalonnage, dans lequel les commandes de séquence d'impulsion d'étalonnage amènent le système d'imagerie à résonance magnétique à acquérir des données de résonance magnétique d'étalonnage de phase en fonction du protocole de thermométrie d'imagerie par résonance magnétique, dans lequel l'exécution des instructions exécutables par machine amène en outre le processeur à :
faire l'acquisition (502) des données de résonance magnétique d'étalonnage de phase en commandant le système d'imagerie à résonance magnétique avec les commandes de séquence d'impulsion d'étalonnage avant commande du système à ultrasons focalisés à haute intensité pour traiter par ultrasons le volume cible actuel ; et
calculer (504) un étalonnage de phase à l'aide des données de résonance magnétique d'étalonnage de phase, dans lequel la carte de température est calculée à l'aide des données de résonance magnétique thermique et de l'étalonnage de phase.

7. Instrument médical selon la revendication 6, dans lequel l'instrument médical comprend en outre un système d'actionneur pour déplacer le transducteur ultrasonique, dans lequel les commandes de traitement par ultrasons spécifient une position d'actionneur pour chaque ensemble de multiples volumes cibles, dans lequel l'exécution des instructions amène en outre le processeur à créer (500) une liste de positions d'actionneur possibles à partir de la position d'actionneur de chaque ensemble de multiples volumes cibles, dans lequel les données de résonance magnétique d'étalonnage de phase sont acquises par acquisition des données de résonance magnétique d'étalonnage de phase pour chaque position d'actionneur dans la liste de positions d'actionneur possibles, dans lequel l'étalonnage de phase est calculé en calculant l'étalonnage de phase pour chaque position d'actionneur dans la liste de positions d'actionneur possibles.

8. Instrument médical selon la revendication 7, dans lequel l'exécution des instructions exécutables par machine amène le processeur à calculer la carte de température pour chaque position d'actionneur dans la liste de positions d'actionneur possibles à l'aide des données de résonance magnétique thermique pour chaque position d'actionneur dans la liste de positions d'actionneur possibles, dans lequel la commande du système à ultrasons focalisés à haute intensité pour traiter par ultrasons la cible actuelle comprend le fait de commander au système d'actionneur de déplacer le transducteur ultrasonique dans la position d'actionneur du volume cible actuel.

9. Produit de programme informatique comprenant des instructions exécutables par machine (180, 182, 184, 186) à exécuter par un processeur (144) commandant un instrument médical (100), dans lequel l'instrument médical comprend un système à ultrasons focalisés à haute intensité (104) comprenant un transducteur ultrasonique ; dans lequel le transducteur ultrasonique comprend de multiples éléments transducteurs pour traiter par ultrasons une zone cible (140), dans lequel le système à ultrasons focalisés à haute intensité peut être utilisé pour diriger électroniquement un emplacement de traitement par ultrasons (138) en commandant l'amenée de courant électrique à chacun des multiples éléments transducteurs, dans lequel l'instrument médical comprend en outre un système d'imagerie à résonance magnétique (102) pour acquérir des données d'imagerie à résonance magnétique thermique provenant d'une zone d'imagerie, dans lequel la zone cible est à l'intérieur de la zone d'imagerie ;
dans lequel l'exécution des instructions exécutables par machine amène le processeur à :
recevoir (300) les commandes de traitement par ultrasons (160), dans lequel les commandes de traitement par ultrasons spécifient un ensemble de multiples volumes cibles (202) à l'intérieur de la zone cible ; et
recevoir (302) une sélection d'un volume cible actuel (200) sélectionné dans le groupe constitué par l'ensemble de multiples volumes cibles ;
dans lequel l'exécution des instructions exécutables par machine amène de façon répétée le processeur à :
acquérir (304) les données de résonance magnétique thermique en commandant le système d'imagerie à résonance magnétique avec des commandes de séquence d'impulsion thermométrique, dans lequel les commandes de séquence d'impulsion thermométrique amènent le système d'imagerie à résonance magnétique à acquérir les données d'imagerie de résonance magnétique thermique en fonction d'un protocole de thermométrie d'imagerie par résonance magnétique ;
calculer (306) une carte de température (168) à l'aide des données de résonance magnétique thermique ;
commander (308) le système à ultrasons focalisés à haute intensité pour traiter par ultrasons le volume cible actuel en dirigeant l'emplacement de traitement par ultrasons vers le volume cible actuel ;
retirer (310) le volume cible actuel hors de l'ensemble de multiples volumes cibles après commande du système à ultrasons focalisés à haute intensité pour traiter par ultrasons le volume cible actuel ;
calculer (312) une énergie de traitement par ultrasons (172) pour chacun des multiples volumes cibles à l'aide de la carte de température ;
sélectionner (314) un volume cible suivant à partir des multiples volumes cibles à l'aide du calcul de l'énergie de traitement par ultrasons pour chacun des multiples volumes cibles ;
**caractérisé en ce que** la section du volume cible suivant comprend la recherche de l'énergie de traitement par ultrasons avec une valeur minimale ; et
définir (316) le volume cible suivant comme le volume cible actuel.
